# EUROPEAN PATENT SPECIFICATION

(11) **EP 1 239 877 B1**
(45) Date of publication and mention of the grant of the patent: **09.01.2008**
(21) Application number: 00969396.1
(22) Date of filing: 02.10.2000
(51) Int. Cl.: A61K 45/06

(54) **COMPOSITION COMPRISING A TNF-ALPHA INHIBITOR AND AN INTEGRIN ALPHAVBETA3 RECEPTOR ANTAGONIST**
ZUSAMMENSETZUNG ENTHALTEND EINEN TNF-ALPHA INHIBITOR UND EINEN INTEGRIN ALPHAVBETA3 REZEPTORANTAGONIST
COMPOSITION COMPRENANT UN INHIBITEUR DU TNF-ALPHA ET D'UN ANTAGONISTE DU RECEPTEUR ALPHAVBETA3 D'INTEGRINE

(30) Priority: 06.10.1999 DE 19948269; 24.12.1999 DE 19962998; 06.06.2000 DE 10027514; 14.06.2000 DE 10028575; 11.08.2000 DE 10039998
(43) Date of publication of application: 18.09.2002
(73) Proprietor: Abbott GmbH & Co. KG, 65205 Wiesbaden (DE)
(72) Inventor: GENESTE, Herve, 67141 Neuhofen (DE); HORNBERGER, Wilfried, 67434 Neustadt (DE)
(74) Representative: Vogelsang-Wenke, Heike
(86) International application number: PCT/EP2000/009673
(87) International publication number: WO 2001/024828

(56) References cited:
- EP-A- 0 922 768
- WO-A-96/06941
- WO-A-99/26945
- WO-A-99/30730
- WO-A-99/31061
- US-A- 5 698 195
- TSAI C ET AL: "Responsiveness of human T lymphocytes to bacterial superantigens presente by cultured rheumatoid arthritis synoviocytes." ARTHRITIS AND RHEUMATISM, vol. 39, no. 1, January 1996 (1996-01), pages 125-136, XP001037860
- ELLIOTT MICHAEL J ET AL: "Randomised double-blind comparison of chimeric monoclonal antibody to tumour necrosis factor alpha (cA2) versus placebo in rheumatoid arthritis" LANCET, LITTLE, BROWM AND CO., BOSTON,, US, vol. 344, no. 8930, 1994, pages 1105-1110, XP002172700 ISSN: 0099-5355
- RANKIN E C C ET AL: "THE THERAPEUTIC EFFECTS OF AN ENGINEERED HUMAN ANTI-TUMOUR NECROSISFACTOR ALPHA ANTIBODY (CDP571) IN RHEUMATOID ARTHRITIS" BRITISH JOURNAL OF RHEUMATOLOGY, BAILLIERE TINDALL, LONDON, GB, vol. 34, no. 4, 1 April 1995 (1995-04-01), pages 334-342, XP000674590 ISSN: 0263-7103
- AREND WP. ET AL: "Review: Inhibition of the production and effects of interleukin-1 and tumor necrosis factor-alpha in rheumatoid arthritis" ARTHRITIS AND RHEUMATISM, vol. 38, no. 2, February 1995 (1995-02), pages 151-160, XP001026695
- STORGARD C M ET AL: "Decreased angiogenesis and arthritic disease in rabbits treated with an alphav-beta3 antagonist" JOURNAL OF CLINICAL INVESTIGATION, vol. 103, no. 1, January 1999 (1999-01), pages 47-54, XP002187023
- MCINNES I B ET AL: "Interleukin-15 mediates T cell-dependent regulation of tumor necrosis factor -alpha production in rheumatoid arthritis." NATURE MEDICINE, vol. 3, no. 2, February 1997 (1997-02), pages 189-195, XP002103447
- ATTUR M G ET AL: "FUNCTIONAL GENOMIC ANALYSIS IN ARTHRITIS-AFFECTED CARTILAGE: YIN-YANG REGULATION OF INFLAMMATORY MEDIATORS BY ALPHA5BETA1 AND ALPHAVBETA3 INTEGRINS" JOURNAL OF IMMUNOLOGY, THE WILLIAMS AND WILKINS CO. BALTIMORE, US, vol. 164, no. 5, 2000, pages 2684-2691, XP001026082 ISSN: 0022-1767

## Description

The invention relates to the use of modulators of an inhibitor of TNFα in combination with integrin αᵥβ₃ receptor antagonists for the treatment or prevention of inflammatory or autoimmune disorders, particularly for the treatment or prevention of rheumatoid arthritis and to the pharmaceutical composition itself.

Rheumatoid arthritis (RA) is a complex chronic inflammatory disease which affects approximately 1 to 3 % of the general population. A variety of anti-inflammatory and immunosuppresive regimens have been employed to limit disease. However, significant toxicity is associated with current therapies which subdue but ultimately fail to stop progression to erosive joint destruction.

It is known, that TNFα, a cytokine produced by numerous cell types, has been implicated in activating tissue inflammation and causing joint destruction in rheumatoid arthritis (see *e.g*., Moeller, A., et al. (1990) Cytokine 2:162-169; U.S. Patent No. 5,231,024 to Moeller et al.; European Patent Publication No. 260 610 B1 by Moeller, A.; WO 9729131; Tracey and Cerami, *supra;* Arend, W.P. and Dayer, J-M. (1995) Arth. Rheum. 38:151-160; Fava, R.A., et al. (1993) Clin. Exp. Immunol. 94:261-266).

On the other hand, it is known that cytokines, for example, IL-10 and Il-4 may have an anti-inflammatory effect. Therefore, it is believed, that compounds that suppress or inhibit proinflammatory cytokine mediated signalling pathways (anti-proinflammatory-cytokine compounds) and compounds that stimulate anti-inflammatory cytokine mediated signalling pathways (anti-inflammatory compounds) may be useful for the treatment of RA (Bredveld, Rheumatology 1999, 38, 11 to 13).

Cheresh et al. describe that suppressors of angiogenesis, such as αᵥβ₃ antagonists, might be useful for the treatment of RA (The Journal of Clinical Investigation 1999, 103, 1, p.47 to 54; Braz. J. Med. Biol. Res. 1999, 32, p. 573 to 581).

It is an object of the present invention to provide an effective pharmaceutical composition for the treatment or prevention of inflammatory or autoimmune disorders, particularly for the treatment or prevention of rheumatoid arthritis, with acceptable side effects and advantageous properties.

We have found that this object is achieved by using TNFα inhibitors in combination with an integrin αᵥβ₃ receptor antagonist.

By combining compounds which act as modulators of cytokine mediated signalling pathways and integrin αᵥβ₃ receptor antagonists either in one formulation or as a kit-of-parts combination by applying both separately via the same or different routes, it is possible to achieve an inhibitory effect on inflammatory pathomechanisms causing rheumatoid arthritis significantly more pronounced than one of the two treatments alone at the given doses. The combination of modulators of cytokine mediated signalling pathways and integrin αᵥβ₃ receptor antagonists in doses too low to be effective alone is as least as effective as a high mono-therapy with either agent and has less potential for side-effects than one principle alone.

Therefore, the invention relates to the use of an inhibitor of TNFα in combination with an integrin αᵥβ₃ receptor antagonist for the manufacture of medicaments for the treatment or prevention of inflammatory or autoimmune disorders, particularly of rheumatoid arthritis.

Inflammatory or autoimmune disorders are, for example, rheumatoid arthritis, rheumatoid spondylitis, osteoarthritis, gouty arthritis, allergy, multiple sclerosis, autoimmune diabetes, autoimmune uveitis or nephrotic syndrome.

In a preferred embodiment, the combination according to the invention can be used for the manufacture of medicaments for the treatment or prevention of rheumatoid arthritis.

Modulators of cytokine mediated signalling pathways result in an anti-inflammatory effect. Therefore, modulators of cytokine mediated signalling pathways are compounds that suppress or inhibit proinflammatory cytokine mediated signalling pathways (anti-proinflammatory-cytokine compounds),
such as, for example, TNFα-inhibitors, particularly TNFa-antibodies, inhibitors of interleukin-1β converting enzyme (ICE inhibitors), inhibitors of Interleukin 1 (IL-1 inhibitors) such as IL-1RA (IL-1 receptor antagonist, Synergen/Amgen), inhibitors of Interleukin 2 (IL-2 inhibitors) such as anti-IL2R antibodies, DAB 486-IL-2 and/or DAB 389-IL-2 (IL-2 fusion proteins, Seragen, see e.g., Arthritis & Rheumatism (1993) Vol. 36, 1223) or Anti-Tac (humanized anti-IL-2Ra, Protein Design Labs/Roche), inhibitors of Interleukin 6 (IL-6 inhibitors), inhibitors of Interleukin 12 (IL-12 inhibitors), inhibitors of Interleukin 17 (IL-17 inhibitors, see *e.g*., Arthritis & Rheumatism (1996) Vol. 39, No. 9 (supplement), S120), inhibitors of Interleukin 18 (IL-18 inhibitors) or antinflammatory or antiautoimmune drugs such as R973401 (phosphodiesterase Type IV inhibitor, see *e.g.,* Arthritis & Rheumatism (1996) Vol. 39, No. 9 (supplement), S282), MK-966 (COX-2 Inhibitor, see *e.g.,* Arthritis & Rheumatism (1996) Vol. 39, No. 9 (supplement), S81), Iloprost (see *e.g*., Arthritis & Rheumatism (1996) Vol. 39, No. 9 (supplement), S82), methotrexate, thalidomide (see *e.g.,* Arthritis & Rheumatism (1996) Vol. 39, No. 9 (supplement), S282) and thalidomide-related drugs (*e.g*., Celgen), leflunomide (anti-inflammatory and cytokine inhibitor, see *e.g*., Arthritis & Rheumatism (1996) Vol. 39, No. 9 (supplement), S131, Inflammation Research (1996) Vol. 45, pp. 103-107), tranexamic acid (inhibitor of plasminogen activation, see *e.g.,* Arthritis & Rheumatism (1996) Vol. 39, No. 9 (supplement), S284), T-614 (cytokine inhibitor, see *e.g*., Arthritis & Rheumatism (1996) Vol. 39, No. 9 (supplement), S282), prostaglandin E1 (see *e.g*., Arthritis & Rheumatism (1996) Vol. 39, No. 9 (supplement), S282), Tenidap (non-steroidal anti-inflammatory drug, see *e.g.,* Arthritis & Rheumatism (1996) Vol. 39, No. 9 (supplement), S280), Na-proxen (non-steroidal anti-inflammatory drug, see *e.g*., Neuro Report (1996) Vol. 1, pp. 1209-1213), Meloxicam (non-steroidal anti-inflammatory drug), Ibuprofen (non-steroidal anti-inflammatory drug), Piroxicam (non-steroidal anti-inflammatory drug), Di-clofenac (non-steroidal anti-inflammatory drug), Indomethacin (non-steroidal anti-inflammatory drug), Sulfasalazine (see *e.g*., Arthritis & Rheumatism (1996) Vol. 39, No. 9 (supplement), S281), Azathioprine (see *e.g.,* Arthritis & Rheumatism (1996) Vol. 39, No. 9 (supplement), S281), zap-70 and/or lck inhibitor (inhibitor of the tyrosine kinase zap-70 or lck), VEGF inhibitor and/or VEGF-R inhibitor (inhibitos of vascular endothelial cell growth factor or vascular endothelial cell growth factor receptor, inhibitors of angiogenesis), corticosteroid anti-inflammatory drugs (*e.g*., SB203580), gold, penicillamine, chloroquine, hydroxychloroquine, chlorambucil, cyclophosphamide, cyclosporine, total lymphoid irradiation, anti-thymocyte globulin, anti-CD4 antibodies, CD5-toxins, collagen, lobenzarit disodium, Cytokine Regulating Agents (CRAs) HP228 and HP466 (Houghten Pharmaceuticals, Inc.), ICAM-1 antisense phosphorothioate oligodeoxynucleotides (ISIS 2302, Isis Pharmaceuticals, Inc.), soluble complement receptor 1 (TP10, T Cell Sciences, Inc.), prednisone, orgotein, glycosaminoglycan polysulphate, minocycline, marine and botanical lipids (fish and plant seed fatty acids, see *e.g*., DeLuca et al. (1995) Rheum. Dis. Clin. North Am. 21:759-777), auranofin, phenylbutazone, meclofenamic acid, flufenamic acid, intravenous immune globulin, zileuton, mycophenolic acid (RS-61443), tacrolimus (FK-506), sirolimus (rapamycin), amiprilose (therafectin), cladribine (2-chlorodeoxyadenosine) or azaribine
or compounds that stimulate anti-inflammatory cytokine mediated signalling pathways (anti-inflammatory compounds)
such as interleukin 4 (IL-4, anti-inflammatory cytokine, DNAX/Schering), interleukin 10 (IL-10, SCH 52000, recombinant IL-10, anti-inflammatory cytokine, DNAX/Schering), interleukin-11 (see *e.g*., Arthritis & Rheumatism (1996) Vol. 39, No. 9 (supplement), S296), interleukin-13 (see *e.g.,* Arthritis & Rheumatism (1996) Vol. 39, No. 9 (supplement), S308) or IL-4-, IL-10-, IL-11 or IL-13 agonists (e.g., agonist antibodies).

Inhibitors are preferred low molecular molecules, antisense molecules or mono or polyclonal antibodies.

Further described as modulators of cytokine mediated signalling pathways are TNFα inhibitors, inhibitors of interleukin-1β converting enzyme (ICE inhibitors) or inhibitors of IL-12 or IL-18, particularly TNFα antibodies.

Also described are ICE inhibitors that are compounds which have a Kᵢ value of 1µM or less, a Kᵢ value of 100nM or less or a Kᵢ value of 10nM or less,
such as L-Alaninamide (N-((phenylmethoxy)carbonyl)-L-valyl-N-((1S)-3-((2,6-dichlorobenzoyl)oxy )-1-(2-ethoxy-2-oxoethyl)-2-oxopropyl), SDZ-224-015, VE-13045, Novartis); 6a,12a-epoxy-1,2,3,4,6a,7,12,12a-octahydro-3,7-dihydroxy-8-methoxy-3-methyl-benz(a)anthracen-1,12-dione (E1-1507-1, E1-1507-2, Kyowa Hakko), VX-740, HMR-3480 (Aventis, Pharmaprojects databases), N-(N-((2S,3S)-3-trans-carboxyoxirane-2-carbonyl)-L-phenylalanyl)-1,4-diaminobutane (TAN-1756A, TAN-1756B, Takeda), (2S-cis)-5-(Benzyloxycarbonylamino-1,2,4,5,6,7-hexahydro-4-(oxoazepino(3,2,1-hi)indole-2-carbonyl)-amino)-4-oxobutanoic acid, Idun (US).

Suitable for the combination of the invention are in principle all TNFα inhibitors, such as TNFα antibodies, TNFα-convertase inhibitors or the compounds SR-31747 (Cyclohexanamine, N-(3-(3-chloro-4-cyclohexylphenyl)-2-propenyl)-N-ethyl-,hydrochloride, (Z)-(CAS), Sanofi-Synthelabo , Pharmaprojects databank), 75 kdTNFR-IgG (75 kD TNF receptor-IgG fusion protein, Immunex; see *e.g.,* Arthritis & Rheumatism (1994) Vol. 37, S295; J. Invest. Med. (1996) Vol. 44, 235A), 55 kdTNFR-IgG (55 kD TNF receptor-IgG fusion protein; Hoffmann-LaRoche), TNF-bp/s-TNFR (soluble TNF binding protein; see *e.g*., Arthritis & Rheumatism (1996) Vol. 39, No. 9 (supplement).

More preferred TNFα inhibitors are TNFα antibodies, for example as described in EP 186833 B1, EP 614984, EP 516785, EP 626389, EP 492488, EP 351789, EP 659766, WO 9429347, EP 701571, EP 486526, WO 9216553, EP 610201, EP 366043, US 5672347, US 5795967, US 5807715, EP 260610 B1 or WO 9729131.

Most preferred TNFα antibodies are poly- or monoclonal, human, humanized, murine or chimeric TNFα antibodies such as CDP-571/Bay-10-3356 (humanized TNFα antibodiy, Celltech/Bayer), cA2 (chimeric TNFα antibody, Centocor), S284; Amer. J. Physiol. - Heart and Circulatory Physiology (1995) Vol. 268, pp. 37-42), D2E7 (WO 9729131, Knoll AG), MAK 195 (EP 260610, BASF Aktiengesellschaft), Synergen (AmgenWorld, Scrip 1997, 2216, 26), Yeda (Ares-Serono, Scrip 1992, 1687, 24), BB-2983 (Glaxo Wellcome, Pharmaprojects database), AGT1 (Advanced Biotherapy Concepts), sTNF-R1 (Amgen, Scrip Daily Online, 22 Nov. 1999) or TNF-484 (Novartis, Pharmaprojects database), particularly D2E7.

Further preferred TNFα antibodies are antibodies, or an antigen-binding portion thereof, that dissociates from human TNFα with a K_{d} of 1 x 10⁻⁸ M or less and a K_{off} rate constant of 1 x 10⁻³ s⁻¹ or less, both determined by surface plasmon resonance, and neutralizes human TNFα cytotoxicity in a standard in vitro L929 assay with an IC₅₀ of 1 x 10⁻⁷ M or less. More preferably, the antibody, or antigen-binding portion thereof, dissociates from human TNFα with a K_{off} of 5 x 10⁻⁴ s⁻¹ or less, or even more preferably, with a K_{off} of 1 x 10⁻⁴ s⁻¹ or less. More preferably, the antibodies, or antigen-binding portion thereof, neutralizes human TNFα cytotoxicity in a standard in vitro L929 assay with an IC₅₀ of 1 x 10⁻⁸ M or less, even more preferably with an IC₅₀ of 1 x 10⁻⁹ M or less and still more preferably with an IC₅₀ of 5 x 10⁻¹⁰ M or less.

A "neutralizing antibody", as used herein (or an "antibody that neutralized hTNFα activity"), is intended to refer to an antibody whose binding to hTNFα results in inhibition of the biological activity of hTNFα. This inhibition of the biological activity of hTNFα can be assessed by measuring one or more indicators of hTNFα biological activity, such as hTNFα-induced cytotoxicity (either *in vitro* or *in vivo*), hTNFα-induced cellular activation and hTNFα binding to hTNFα receptors. These indicators of hTNFα biological activity can be assessed by one or more of several standard in *vitro* or *in vivo* assays known in the art. Preferably, the ability of an antibody to neutralize hTNFα activity is assessed by inhibition of hTNFα-induced cytotoxicity of L929 cells. As an additional or alternative parameter of hTNFα activity, the ability of an antibody to inhibit hTNFα-induced expression of ELAM-1 on HUVEC, as a measure of hTNFα-induced cellular activation, can be assessed.

The term "surface plasmon resonance", as used herein, refers to an optical phenomenon that allows for the analysis of real-time biospecific interactions by detection of alterations in protein concentrations within a biosensor matrix, for example using the BIAcore system (Pharmacia Biosensor AB, Uppsala, Sweden and Piscataway, NJ). For further descriptions, see Example 1 and Jönsson, U., et al. (1993) Ann. Biol. Clin. 51:19-26; Jönsson, U., et al. (1991) Biotechniques 11:620-627; Johnsson, B., et al. (1995) J. Mol. Recognit. 8:125-131; and Johnnson, B., et al. (1991) Anal. Biochem. 198:268-277.

The term "K_{off}", as used herein, is intended to refer to the off rate constant for dissociation of an antibody from the antibody/antigen complex.

The term "K_{d}", as used herein, is intended to refer to the dissociation constant of a particular antibody-antigen interaction.

Preferred integrin αᵥβ₃ receptor antagonists within the scope of the invention are substances which show an IC₅₀ value of 100nM or less for the inhibition of vitronectin binding to integrin αᵥβ₃ in an ELISA assay, which is, described for example in DE 19919218.9 (German application number). Suitable integrin αᵥβ₃ receptor antagonists for the combination of the invention are, in principle, all integrin αᵥβ₃ receptor antagonists, for example as described in Pitts et al.; J. Med. Chem. 2000, 43, 27-40; Batt et al., J. Med. Chem. 2000, 43, 41-51; Miller et al., Bioorg. Med. Chem. Lett. 9, 1999, 1807-1812; Keenan et al., Bioorg. Med. Chem. Lett. 9, 1999, 1801-1806; Rockwell et al., Bioorg. Med. Chem. Lett. 9, 1999, 937-942; Samanen et al., Current Pharm. Design 1997, 3, 545-584; Miller et al., J. Med. Chem. 2000, 43, 22-26; Hartmann and Duggan, Exp. Opin. Invest. Drugs 2000, 9 (6), 1281-1291; Miller et al., Drug Discovery Today 2000, 5 (9), 397-408; DE 19919218.9 (German application number), DE 19948269.1 (German application number), DE 19962998.6 (German application number), DE 10027514.1 (German application number), DE 10028575.9 (German application number), DE 10039998.3 (German application number), WO 9952879, WO 9835917, WO 0000486, WO 0017197, WO 0031067, WO 9843962, WO 9926945, WO 9950249, WO 9958162, WO 0000481, US 6056958, WO 43787, WO 9637492, WO 9723480, WO 9733887, WO 9748395, WO 9748444, WO 9823608, US 5,849,736, DE 19626701, EP 0796855A1, DE 19653645, DE 19653646, DE 19653647, EP 796855, EP 820988, EP 820991, EP 853084, EP 854145, US 5990145, WO 9915506, WO 9915507, WO9932457, WO 9937621, WO 9959992, EP 928790, EP 928793, US 6001855, WO 00024724, WO 9825892, WO 9965944, WO 0048603, WO 9938849, WO 9952872, DE 19534016, DE 19548709, DE 19653036, DE 19654483, DE 19705450, DE 1971300, DE 19725368, DE 19842415, DE 19850131, EP 683173, EP 710657, EP 741133, EP 771 818, WO 9714716, WO 9723451, WO 9738009, WO 9744333, WO 9800395, WO 9818764, wo 9827112, WO 9835949, WO 9901472, WO 9910371, WO 9931126, WO 0003973, WO 0026212, WO 9532710, WO 9726250, WO 9737655, WO 9808518, WO 9808840, WO 9818460, WO 9818461, WO 9831359, WO 9844797, WO 9846220, WO 9901472, WO 9930709, WO 9930713, WO 9931061, WO 9931099, WO 0006169, WO 0009503, US 5981546, US 6017925, US 6017926, WO 9967230, WO 9734865, FR 2768734-Al, FR 2768736-Al, WO 0032578, US 5639765, US 5681820, US 5852210, US 5972986, US 6013651, WO 9708145, WO 9736858, WO 9736859, WO 9736860, WO 9736861, WO 9736862, WO 9944985, WO 9944994, WO 9951638, WO 9952896, WO 0009143, WO 0038665, WO 0038715, WO 0038719, WO 0038786, WO 9600574, WO 9600730, WO 9606087, WO 9626190, WO 9701540, WO 9724119, WO 9724122, WO 9724124, WO 9724336, WO 9814192, WO 9815278, WO 9829561, WO 9830542, WO 9840488, WO 9905107, WO 9906049, WO 9911626, WO 9915170, WO 9915178, WO 9915508, WO 9945927, WO 0007544, WO 0033838 or WO 9933798, particularly, the following proteins, peptidic and nonpeptidic compounds.

Proteins and peptidic integrin αᵥβ₃ receptor antagonists:
LM 609 (vitaxin, Pharmaprojects),
abciximab (c7E3 Fab, Reopro®, Pharmaprojects),
Peptides and peptidomimetics of Arg-Gly-Asp and derivatives thereof like:
   cyclo(RGDfV), As-Pen-RGDC-OH, cyclo[RGD-Mamb-P], XJ 735 (cyclo[R-G-D-Mamb-A]), XK 002 (cyclo[(NMe)R-G-D-(2-amino-1,3-thiazol-4yl-acetic acid)-V]), DMP 728 (cyclo[[(NMe)R-G-D-Mamb-DAbu]), SK+F 107260 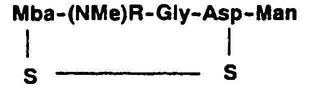
EMD 121974 (cyclo[R-G-D-f-(NMe)V]) and any other RGD containing peptides.

Non-peptidic integrin αᵥβ₃ receptor antagonists: (2*R*)-2-[((2*R*)-2-{3-[(3-{[amino(imino)methyl]amino}propanoyl)amino]phenyl}-3-carboxy propanoyl)amino]-3-methylbutanoic acid, 3-[8-(2-{[amino(imino)methyl]amino}ethyl)-1-benzyl-2-oxo-1,2,3,5-tetrahydro-4*H*-1,4-benzodiazepin-4-yl]propanoic acid, 2,3-dihydroxypropyl 2-{[(benzyloxy)carbonyl]amino}-4-({9,10-dimethoxy-4-[(*E*)-2-(1,4,5,6-tetrahydropyrimidin-2-yl)hydrazono]-1,2,3,3a,4,5,6,10b-octahydrobenzo[*e*]azulen-8-yl}oxy)butanoate, (2*S*)-2-{[(benzyloxy)carbonyl]amino}-3-[({(4*S*)-4-[3-(4,5-dihydro-1*H*-imidazol-2-ylamino)propyl]-2,5-dioxoimidazolidin-1-yl}acetyl)amino]propanoic acid, L-7418415 ((2*S*)-2-[(phenylsulfonyl)amino]-3-({4-[2-(1,4,5,6-tetrahydropyrimidin-2-ylamino)ethoxy]benzoyl}amino)propanoic acid), (2*S*)-2-{[(4-isobutylphenyl)sulfonyl]amino}-3-[({5-[3-(pyridin-2-ylamino)propyl]-4,5-dihydroisoxazol-3-yl}carbonyl)amino]propanoic acid, (2*S*)-2-{[(benzyloxy)carbonyl]amino}-3-[({4-[4-(4,5-dihydro-1*H*-imidazol-2-ylamino)butanoyl]piperazin-1-yl}carbonyl)amino]propanoic acid, (2*S*)-2-{[(benzyloxy)carbonyl]amino}-3-[({4-[4-(4,5-dihydro-1*H*-imidazol-2-ylamino)propanoyl]piperazin-1-yl}carbonyl)amino]propanoic acid, SD-186 ((2*S*)-2-[(phenylsulfonyl)amino]-3-[{(8-(pyridin-2-ylamino)methyl}-1-oxa-2-aza-spiro[4.5]dec-2-en-3-yl]carbonyl)amino]propionic acid), SD-183 ((2S)-2-[(phenylsulfonyl)amino]-3-[({8-[(pyridin-2-ylamino)methyl]-1-oxa-2-azaspiro[4.5]dec-2-en-3-yl}carbonyl)-amino]propanoic acid, SD-983 ((2S)-2-{[(benzyloxy)carbonyl]amino}-3-[({3-[3-(4,5-dihydro-1*H-*imidazol-2-ylamino)propoxy]isoxazol-5-yl}carbonyl)amino]propanoic acid), XT-199 ((2*S*)-3-[({3-[3-(4,5-dihydro-1*H*-imidazol-2-ylamino)propoxy]isoxazol-5-yl}carbonyl)amino]-2-[(phenylsulfonyl)amino]propanoic acid), SG-545 (Methyl (2*S*)-2-{[(benzyloxy)carbonyl]amino}-3-[({3-[3-(4,5-dihydro-1*H*-imidazol-2-ylamino)propoxy]isoxazol-5-yl}carbonyl)amino]propanoic acid), SM 256 ((2*S*)-3-[({1-[3-(1*H*-imidazol-2-ylamino)propyl]-1*H*-indazol-5-yl}carbonyl)amino]-2-[(mesitylsulfonyl)amino]propanoic acid), SD-836 (Pharmaprojects), SD-7784 (Pharmaprojects), SD-7783 (Pharmaprojects), S-137 (N-({[1-(4-{[amino(imino)methyl]amino}butyl)vinyl]amino}acetyl)-3-pyridin-3-yl-beta-alanine), S-787 (Seattle et al.; 21st Ann. Meet. Amer. Soc. Bone Mineral Res., 30.9.-4.10.1999; SU 410), S 448 (N-{[(3-{[amino(imino)methyl]amino}benzoyl)amino]acetyl}-3-phenyl-β-alanine), SC 68448 (N-{[(3-{[amino(imino)methyl]amino}benzoyl)amino]acetyl}-3-(3,5-dichlorophenyl)-β-alanine), SC 56631 (N-{[(5-{[amino(imino)methyl]amino}pentanoyl)amino]acetyl}-3-pyridin-3yl-β-alanine), SC 69000 (4-[(3-{[amino(imino)methyl]amino}benzoyl)amino]-*N*-(isobutoxycarbonyl)phenylalanine), SC-65811 (*N*-{[(3-{[(benzylamino)carbonyl]amino}benzoyl)amino]acetyl}-3-pyridin-3-yl-b-alanine), SB 223245 (((2*S*)-7-{[(1*H*-benzimidazol-2-ylmethyl)(methyl)amino]carbonyl}-4-methyl-3-oxo-2,3,4,5-tetrahydro-1*H*-1,4-benzodiazepin-2-yl)acetic acid), SB 265123 ([(10S)-3-[3-(pyridin-2yl-amino)propoxy]-10,11-dihydro-5H-dibenzo[a,d]-cyclohepten-10yl]acetic acid), SB 267268 ([(4S)-3-oxo-8-[3-(pyridin-2-ylamino)propoxy]-2-(2,2,2-trifluoroethyl)-2,3,4,5-tetrahydro-1*H*-2-benzazepin-4-yl]acetic acid), SB 273005 (Lark et al.; 21st Ann. Meet. Amer. Soc. Bone Mineral Res., 30.9.-4.10.1999; SU201), CP-4632 ((2*S*)-3-[(3-fluoro-4-[4-(1,4,5,6-tetrahydropyrimidin-2ylamino)piperidin-lyl]benzoyl)amino]-2-[(phenylsulfonyl)amino]propanoic acid), (2*S*)-3-({3-chloro-4-[4-(1,4,5,6-tetrahydropyrimidin-2-yl)piperidin-1-yl]benzoyl}amino)-2-[(phenylsulfonyl)amino]propanoic acid), SH306 (2S)-2-[(mesitylsulfonyl)amino]-3-[({1-[3-(pyridin-2-ylamino)propyl]-1H-indazol-5-yl}carbonyl)amino]propanoic acid, SB 273005 (Lark et al.; 21st Ann. Meet. Amer. Soc. Bone Mineral Res., 30.9.-4.10.1999; SU201) [(4S)-8-{2-[6-(Methylamino)pyridin-2-yl]ethoxy}-3-oxo-2-(2,2,2-trifluoroethyl)-2,3,4,5-tetrahydro-1*H*-2-benzazepin-4-yl]acetic acid, SC 72115 (3-(5-bromo-3-chloro-2-hydroxyphenyl)-N-({[3-(4,5-dihydro-1H-imidazol-2-ylamino)benzoyl]amino}acetyl)-beta-alanine).

Preferred are non-peptidic antagonists, particularly those which are orally available and integrin αᵥβ₃ receptor antagonists selected from the group:
LM 609 (vitaxin), EMD 121974 (cyclo[R-G-D-f-(NMe)V]), L-7418415 ((2S)-2-[(phenylsulfonyl)amino]-3-({4-[2-(1,4,5,6-tetrahydropyrimidin-2-ylamino)ethoxy]benzoyl}amino)propanoic acid), SB 265123 ([(10S)-3-[3-(pyridin-2yl-amino)propoxy]-10,11-dihydro-5H-dibenzo[a,d]-cyclohepten-10yl]acetic acid), SB 267268 ([(4*S*)-3-oxo-8-[3-(pyridin-2-ylamino)propoxy]-2-(2,2,2-trifluoroethyl)-2,3,4,5-tetrahydro-1*H*-2-benzazepin-4-yl]acetic acid), SB 273005 (Lark et al.; 21st Ann. Meet. Amer. Soc. Bone Mineral Res., 30.9.-4.10.1999; SU201), SC 68448 (N-{[(3-{[amino(imino)methyl]amino}benzoyl)amino]acetyl}-3-(3,5-dichlorophenyl)-β-alanine), SC 69000 (4-[(3-{[amino(imino)methyl]amino}benzoyl)amino]-*N*-(isobutoxycarbonyl)phenylalanine and SC-65811 (*N*-{[(3-{[(benzylamino)carbonyl]amino}benzoyl)amino]acetyl}-3-pyridin-3-yl-b-alanine).

All mentioned compounds can also be used as prodrugs. Prodrugs are substances which metabolise in vivo to the active compound. Examples for such metabolism are first pass metabolisms (e.g. esters to free acids or carboxylates).

"Orally available" means at least 10%, preferred 30% and more preferred 50% for integrin αᵥβ₃ receptor antagonist.

All mentioned compounds may be used as such or in the form of their salts with physiologically tolerated acids or bases. Antibodies may also be used as antibody-portions.

Preferred combinations of TNFα inhibitors with integrin αᵥβ₃ receptor antagonists are selected from the preferred TNFα inhibitors and the preferred integrin αᵥβ₃ receptor antagonists.

The TNFα inhibitors in combination with the integrin αᵥβ₃ receptor antagonist may be used together in a pharmaceutical composition or they may be used for the manufacture of a medicament suitable to be administered simultaneously via separate ways or separately or temporally graduated.

Therefore, the invention further relates to a pharmaceutical composition, comprising an inhibitor of TNFα and an integrin αᵥβ₃ receptor antagonist.

This composition can be used as a medicament, particularly for curing or preventing inflammatory or autoimmune disorders, such as rheumatoid arthritis, rheumatoid spondylitis, osteoarthritis, gouty arthritis, allergy, multiple sclerosis, autoimmune diabetes, autoimmune uveitis or nephrotic syndrome.

In a preferred embodiment, the composition is used for the treatment or prevention of rheumatoid arthritis.

The composition of the invention can be administered orally or parenterally in a conventionally way (subcutaneously, intravenousely, intramusculary, intraperitoneally, rectally). Administration can also take place with vapours or sprays through the nasopharyngeal space. Oral administration is preferred.

The dosage depends on age, condition and weight of the patient and on the mode of administration. The two compounds can be formulated in a single pharmaceutical form or in separate pharmaceutical forms. Administration can be given in several single doses (e.g. 2 to 4) or once or twice a day as depot form.

The weight ratio of integrin aᵥβ₃ receptor antagonist to modulators of cytokine mediated signalling pathways conveniently is in the range of 1:100 to 100:1 preferably 1:10 to 10:1. Advantageously, the dosage to be administered by means of a combination per day and kg amounts to 0,05 to 20 mg of an integrin αᵥβ₃ receptor antagonist and 0,1 to 20 mg, preferably 1 to 10 mg of an modulator of cytokine mediated signalling pathways. In general, the total amount of an integrin αᵥβ₃ receptor antagonist and an modulators of cytokine mediated signalling pathways to be administered daily amounts per kg to a maximum of 50 mg. When a hydrate or a pharmaceutically usable salt is used, then the above values are to be appropriately adjusted.

The compounds can be used individually or together in conventional solid or liquid pharmaceutical forms, e.g. as uncoated or (film-)coated tablets, capsules, powders, granules, suppositories, solutions, ointments, creams or sprays. These are produced in a conventional way. In these, the active substances can be processed with conventional pharmaceutical aids such as tablet binders, fillers, preservatives, tablet disintegrants, flow regulators, plasticizers, wetting agents, dispersants, emulsifiers, solvents, release slowing agents, antioxidants
and/ or propellant gases (cf. H. Sucker et al. Pharmaceutische Technologie, Thieme Verlag, Stuttgart, 1978). The administration form obtained in this way normally comprises the active substance in an amount of from 0.1% to 99% by weight.

Treatment of a patient with an inflammatory or autoimmune disease by a composition according to the present invention may include concomitant use of further adjunctive agents, such as antiinflammatory drugs as described above.

Subject of the present invention are also pharmaceutical compositions, comprising anintegrin αᵥβ₃ receptor antagonist in an appropriate container and an inhibitor of TNFα in a separate container to be used according to the above-mentioned administration regiments.

Pharmaceutical kits prepared in accordance with the present invention may consist of an appropriate administration form comprising the integrin αᵥβ₃ receptor antagonist, and an appropriate packaging unit comprising the TNFα inhibitor. The two active compounds are preferrably present in the packaging unit in two different containers, e.g. tablets. However, depending on the type of active compound, it may also be possible to provide both compounds in a single dosage form. Further, the pharmaceutical kits comprise instructions, for example in the form of a package leaflet prescribed for medicaments from which it follows that the administration of a therapeutically active amount of the integrin αᵥβ₃ receptor antagonist advantageously takes place in combination with administration of a TNFα inhibitor.

If applied separately, the administration of the TNFα inhibitor takes places before, simultaneously or after the administration of the integrin αᵥβ₃ receptor antagonist.

Information regarding the manner of use can either be given in the information leaflet or as a packing overprint on the medical preparation which can be bought together with medicinal preparations which comprise integrin αᵥβ₃ receptor antagonists. On the one hand, pharmaceutical kits comprising only appropriate administration forms of the integrin αᵥβ₃ receptor antagonists can comprise such information e.g. in the form of package leaflets, wherein the combined administration together with modulators of cytokine mediated signalling pathwayss according to the present invention is mentioned. On the other hand, kits comprising only TNFα inhibitor can comprise such information wherein the combined administration together with integrin αᵥβ₃ receptor antagonists and the use according to the present invention is mentioned. A third alternative would be to provide kits comprising an integrin αᵥβ₃ receptor antagonist, TNFα inhibitor and an appropriate information about the combined use of both, e.g. the usual package leaflet.

Therefore, the invention further relates to a pharmaceutical kit, comprising as pharmaceutical agent a TNFα inhibitor or/and an integrin αᵥβ₃ receptor antagonist together with an instruction for use of this pharmaceutical agents in combination for simultaneous, separate, or temporal graduated application for the treatment or prevention of diseases.

Appropriate directions of use of the above-mentioned pharmaceutical agents are essential for commercialization of such pharmaceutical kits, comprising either the integrin αᵥβ₃ receptor antagonist, the TNFα inhibitor or a combination thereof.

Commercialization of appropriate pharmaceuticals by pharmaceutical companies is only possible when prior approval of such pharmaceutical agents and the respective administration regimens is achieved by the respective national Health Authorities, such as the FDA in the US or the CPMP Authority in Europe.

This includes but is not limited to performing clinical trials according to well-established procedures under the supervision of said pharmaceutical company which lateron intends to commercialize such pharmaceutical agents. This also includes filing of appropriate documentation about the results of such clinical trials with the respective Health Authority in order to get marketing approval. The approval is in many cases restricted to certain administration protocols or regimens which have to be included in printed form in the accompanying information leaflet prescribed for medicaments.

### Examples

### Example 1

### Polyarthitis model in Tg197 transgenic mice

Transgenic mice (Tg197), which have been shown to express human wild type TNFα (modified in the 3' region beyond coding sequences) develop chronic polyarthritis with 100 % incidence at 4 - 7 weeks of age (See WO 9729131, Example DIII).

Transgenic mice are first identified by PCR at 3 days of age and then are verified by by slot blot hybridization analysis at 15 days of age. From the first week of age, litters of transgenic mice are divided into groups of 8 animals each. Before the first weekly injection, average body weight are determined by weighing, all animals in each group and calculating the average body weight. The date and weights of all animals in each group are recorded once a week in the log book.

Each group receive one i.p. injection of a TNFα inhibitor, for example a TNFα antibody, for example D2E7 (see WO 9729131) (dose range 0.1 - 10 µg/g) or per week or a oad dose (i.v., s.c. or oral) of an integrin αvβ3 antagonist or a combination of both compounds/administrations or vehicle.

The treatment protocols for the six groups are as follows:
Group 1=no treatment;
Group 2=saline (vehicle);
Group 3=TNFα inhibitor, for example a TNFα antibody, for example D2E7 ;
Group 4=integrin αVβ3 antagonist;
Group 5, 6=TNFα inhibitor, for example a TNFα antibody, for example D2E7 in combination with integrin αVβ3 antagonist in different dosages;

A litter with non transgenic mice is also included in the study to serve as a control (Group 7 - nontransgenic; no treatment)

Macroscopic changes (in units of arthritic scores) in joint morphology are recorded weekly for each animal. Arthritic scores were recorded as follows; 0 = No arthritis, (normal appearance and flexion); + = mild arthritis joint distortion); ++ = moderate arthritis (swelling, joint deformation) and +++ = heavy arthritis (ankylosis detected on flexion and severely impaired movement).

Sera are collected from 4 out of 8 mice per group by orbital sinus bleeding at 5 weeks of age. At completion of the study all animals are sacrificed and sera are collected by cardiac puncture and stored at -70 °C.

Treatment is continued for 8 weeks. At 9 weeks of age, all mice are sacrificed and ankle joints are collected in formalin. Ankle joint sections were then stained with haematoxylin/eosin and histopathology scores are evaluated microscopically in a series of sections. Histopathological scoring based on haematoxylin/eosin staining of joint sections is based as follows; 0 = No detectable disease; 1 proliferation of the synovial membrane; 2 = heavy synovial thickening, 3 = cartilage destruction and bone erosion.

Levels of integrin αVβ3 antagonists are determined by HPLC. Levels of TNFα inhibitor, for example a TNFα antibody, for example D2E7 are determined by EIA according to the validated PK assay (MPF/EB 9644) with one modification. Biotinylated MAK195F is used instead of biotinylated D2E7 in order to eliminate the interference from murine anti-human antibodies. Levels of murine anti human antibodies (MAHA) are determined in a direct ELISA. Microtiter plates were coated with 10 µg/ml of LU 200134 overnight at 4 °C, and blocked with 3 % teleostean gelatin (Sigma, Cat # G7765) for 2 hours at 25 °C. Diluted serum samples or a standard mouse anti-human antibody (Sigma, Cat # M-9035 ) are added to the plates and incubated overnight at 4 °C. Biotinylated D2E7 at 5 nM is added and incubated for 2 hours at 4 °C. Plates are washed 5 times with PBS between each step. Avidin coupled alkaline phosphatase (Boehringer Mannheim) are added at 115000 dilution and incubated for 1 hour at 4 °C. Bound avidin-alkaline phosphatase is measured with an enzyme amplification kit (TMB, Pierce, Cat # 1854050) according to manufacturer's instructions. ODs are recorded at 490 nm, and the levels of MAHA are assigned from the standard curve.

Statistics: Weekly measurements of weight and ankle joint sizes are recorded for each animal in every group as Excel worksheets. Groups 1 and 7 are compared separately with other groups by two-tailed Student's t-Test. Group 1 and group 7 represent the untreated disease and untreated disease-free control animals, respectively. t-Test function in the Microsoft Excel software was used to obtain probability (P) values of similarity between two groups of experimental animals. The option of two-sample unequal variance was chosen in the t-Test function.

ED₅₀ calculations: For each week, means and standard errors of arthritic scores are plotted as a function of dose. ED₅₀ values are calculated with a non-linear four parameter curve fitting. Histopathological scores determined after the mice have been sacrificed at week 9 are also plotted as a function of dose and ED₅₀ value is derived similarly.

The use of the combination of a modulator of cytokine mediated signalling pathways and an integrin αᵥβ₃ receptor antagonists achieves an inhibitory effect on inflammatory pathomechanisms causing rheumatoid arthritis significantly more pronounced than one of the two treatments alone at the given doses. The combination of a modulator of cytokine mediated signalling pathways and an integrin αᵥβ₃ receptor antagonists in doses too low to be effective alone is effective as a high mono-therapy with either agent and has less potential for side-effects than one principle alone.

## Claims

1. Pharmaceutical composition, comprising an inhibitor of TNFα and an integrin αᵥβ₃ receptor antagonist.

2. Composition according to claim 1 for use as a medicament.

3. Composition according to claim 2 for preventing or treating inflammatory disorders, autoimmune disorders or rheumatoid arthritis.

4. Use of an inhibitor of TNFα in combination with an integrin αᵥβ₃ receptor antagonist for the manufacture of a medicament for the prevention or treatment of inflammatory disorders, autoimmune disorders or rheumatoid arthritis.

5. Kit for the prevention or treatment of inflammatory disorders, autoimmune disorders or rheumatoid arthritis, comprising:
a) an inhibitor of TNFα and/or an integrin αᵥβ₃, receptor antagonist with
b) an instruction for use of these pharmaceutical agents in combination for simultaneous separate, or temporally graduated application.

## Patentansprüche

1. Pharmazeutische Zusammensetzung, umfassend einen Hemmstoff von TNFα und einen Integrin-αᵥβ₃-Rezeptorantagonisten.

2. Zusammensetzung gemäß Anspruch 1 zur Verwendung als Medikament.

3. Zusammensetzung gemäß Anspruch 2 zum Verhindern oder Behandeln von inflammatorischen Erkrankungen, Autoimmunerkrankungen oder rheumatoider Arthritis.

4. Verwendung eines Hemmstoffs von TNFα in Kombination mit einem Integrin-αᵥβ₃-Rezeptorantagonisten zur Herstellung eines Medikaments zur Verhinderung oder Behandlung von inflammatorischen Erkrankungen, Autoimmunerkrankungen oder rheumatoider Arthritis.

5. Kit zur Verhinderung oder Behandlung von inflammatorischen Erkrankungen, Autoimmunerkrankungen oder rheumatoider Arthritis, umfassend:
a) einen Hemmstoff von TNFα und/oder einen Integrin-αᵥβ₃-Rezeptorantagonisten mit
b) einer Anleitung zur Verwendung dieser pharmazeutischen Wirkstoffe in Kombination zur simultanen, separaten oder zur zeitlich abgestuften Anwendung.

## Revendications

1. Composition pharmaceutique comprenant un inhibiteur de TNFα et un antagoniste de récepteur d'intégrine αᵥβ₃.

2. Composition selon la revendication 1 destinée à être utilisée comme médicament.

3. Composition selon la revendication 2 pour prévenir ou traiter les affections inflammatoires, les affections autoimmunes ou la polyarthrite rhumatoïde.

4. Utilisation d'un inhibiteur de TNFα en combinaison avec un antagoniste de récepteur d'intégrine αᵥβ₃ pour la fabrication d'un médicament pour la prévention ou le traitement des affections inflammatoires, des affections autoimmunes ou de la polyarthrite rhumatoïde.

5. Kit pour la prévention ou le traitement des affections inflammatoires, des affections autoimmunes ou de la polyarthrite rhumatoïde, comprenant :
a) un inhibiteur de TNFα et/ou un antagoniste de récepteur d'intégrine αᵥβ₃ avec
b) une instruction pour l'utilisation de ces agents pharmaceutiques en combinaison pour l'application séparée simultanée ou temporellement progressive.
